# EUROPEAN PATENT APPLICATION

(11) **EP 4 074 897 A1**
(43) Date of publication of application: **19.10.2022**
(21) Application number: 20898003.7
(22) Date of filing: 10.12.2020
(51) Int. Cl.: E02B 15/00, B63B 35/32, A01D 44/00, C02F 3/32, C10L 3/06

(54) **VESSEL AND METHOD FOR CONTROLLING AQUATIC PLANT SPECIES**

(30) Priority: 10.12.2019 ES 201932011 U
(71) Applicant: Villalba Hernández, Manuel, 50580 Vera de Moncayo Zaragoza (ES); Villalba Hernández, Andrés, 50009 Zaragoza (ES)
(72) Inventor: CAPUZ BARRIO, Alejandro Alberto, 46540 El Puig (Valencia) (ES); VILLALBA HERNÁNDEZ, Manuel, 50580 Vera de Moncayo (Zaragoza) (ES); VILLALBA HERNÁNDEZ, Andrés, 50009 Zaragoza (ES)
(74) Representative: Sahuquillo Huerta, Jesús
(86) International application number: PCT/ES2020/070779
(87) International publication number: WO 2021/116523

(57) **Abstract**

A vessel for control of aquatic plant species (100) in seas, rivers and lakes that is configured to extract from the surface of the water and hoist to a deck of the vessel (100) a plurality of plant remains of a given aquatic ecosystem by means of an elevator belt (101); and/or receive from a coastal installation a plurality of aquatic plant remains mixed with grey water from coastal human settlements; and that is characterized in that the remains of aquatic plant species extracted from the surface of the water and/or received from coastal human settlements are deposited in a cooking chamber (102) where they are subjected to controlled temperature conditions configured to modify, ferment and rot said remains preventing their reproduction.

## Description

### Technical field of the invention

The present invention relates to a vessel and a method of control of aquatic plant species in seas, rivers, and lakes. The object of the vessel of the invention is a vessel capable of collecting on its own and processing by sterilizing aquatic plant species that may be harmful as they have become a plague, modifying the original ecosystem.

### Background art

It is known that global warming and the overuse of fertilizers used in new intensive agriculture favours the growth of plant species that develop in the water and float, which has led to considerable plague problems. The increase in these species has been much more than exponential and, from something anecdotal has become a serious problem of water health. This situation has ended up being a problem in some areas with substantial economic value, for example in coastal tourist areas.

Faced with this new and pressing need, a multitude of solutions have been adopted that have proved to be clearly ineffective and insufficient, such as:
1.- Floating barriers to prevent passage. These are moved by the wind, and the invasive species end up passing under or over them. This type of floating barrier includes the development of the Dutch firm Ocean Cleanup B.V. described in WO2018208159A1.
2.- Manual removal. Currently, work is being done manually with crews of workers who work on the beach, collecting sand in addition to the algae, which degrades the beach.
3.- Harvesters of algae and water lilies. They are simply vessels that collect small quantities and are not suitable for places with either little or substantial draught and waves, and do not work properly where the distance between the collection point and the discharge point is far away. There is also the circumstance that once unloaded, as the amount they collect each time is small, it is necessary to have mechanized means to remove it from the shore and process it properly, needing a digger, a transport truck and an appropriate landfill in a remote place where the odors and the leachates generated by rotting will not be a nuisance. In addition, it should be noted that uncontrolled offshore rotting of plant debris generates methane gas, which is harmful with respect to greenhouse gas emissions.
4.- Collecting them by mechanical means when they reach the shore and removing them by manual or mechanized means, taking them to a landfill as that described in the previous point.

The enormous drawback of the current means is that the access of sargassums or invasive species to the beaches occurs in a highly or completely unpredictable way, so it is practically impossible to adapt the logistics necessary for the removal of these plants at the same time that it occurs, usually during low tide hours. In addition, if the removal is delayed, the process of fermentation and rotting begins, generating leachates that become a nuisance due to their odor and which colour the beach sand brown.

Another damage caused by the removal of algae or sargassums from the beach is that it is almost impossible not to remove small amounts of sand each time, such that in areas of high tourist potential, the quality of the beach deteriorates.

Document ES1187084U describes a vessel that, on its own, cuts, extracts and crushes in small fragments and throws the plant remains to one side of the channel or reservoir, being able to evacuate them mixed with water through a tube to the ground, so that they are filtered there for subsequent treatment and recycling, or throws them back into the water once they have been chopped into very small fragments, being able to vary the size of the chopped material at the operator's will and no longer representing a visual or landscape nuisance, nor interfering or hindering the opening of gates. The difficulty of this system is that it only serves efficiently at small distances from the beach and provided that between the collection point and the discharge point there are no points that protrude from the surface, such as reefs and corals. It is also necessary to have a point enabled for unloading and subsequent removal to a suitable landfill.

Document CR20200091A describes a vessel intended for the rehabilitation of native marine ecosystems and social integration, with spaces for research, education and productive chains in coastal locations, positioning it as a focus of scientific and business tourism. The vessel is characterised by being modular, floating, habitable and self-sustaining, for the intensive production of marine crops, using highly productive techniques with very low environmental impact; and which will be able to take advantage of photovoltaic, photothermal or marine kinetic energy. Its design allows the collection and treatment of rainwater, the desalination of sea water and its eventual storage. The purpose of this document is to create housing, administrative or laboratory spaces related to hydroponics, mariculture or environmental monitoring, as well as spaces for marine science education, ecotourism services and business support.

Document ES1252114U describes a modular container device for electrical and thermal biocogeneration consisting of a container module of standard dimensions easy to transport by truck, boat or any other means and which is characterized by comprising a biogas-operated turbine or microturbine and an electrical generator, an electrical transformer and a whole series of auxiliary elements for the correct use, control and maintenance thereof, such as doors, ventilation, control panel and safety elements. In addition, this device comprises a series of inlet and outlet connections such as the biogas inlet and as an outlet it comprises the electrical current outlet itself from the electrical transformer of the container.

Document ES 2725606T3 relates to floating lakes and water treatment in such lakes. In addition, this document refers to large floating lakes installed within a body of natural or artificial water. The dimensions of the floating lake, including the depth and surface area of the floating lake, may vary depending on the need and existing resources, as well as the surface area and other physical characteristics of the water body. The floating lake may be provided with a system for applying chemicals; a filtration system including a mobile suction device and filters; and a foaming system; and may also comprise a coordination system. The system and method of the present invention may be configured to produce significant cost savings due to capital investment, energy consumption and lower use of chemicals than conventional systems. This is due to the activation of the methods of the present invention based on the actual requirements of the water body, by the evaluation of specific variables and also due to the fact that it is not necessary to comply with as many rules on redox potential as compared to conventional pool treatment systems, because of the use of an efficient filtration system based on the background colour of the floating lakes.

### Description of the invention

The object of the present invention is a vessel and a method of controlling aquatic plant species in seas, rivers and lakes. The object of the present invention is to attack the plant species by extracting them from the surface of the water or from the beach or coast where they have been established, crushing them and subjecting them - inside the vessel - to the temperature necessary so that, by the action of the heat and the fermentation caused by said heat, they die and their reproduction is impossible. As an advantageous effect of the fermentation and putrefaction process of the collected vegetable residues, it is possible to generate two especially useful by-products: feed for wild fish or fish in farms, and methane gas, which can be used in the processes necessary to accelerate fermentation and putrefaction, as well as the electrical cogeneration in the boat itself.

Another object of the present invention is that the gas generated during the fermentation and rotting of the vegetable waste collected by the vessel can be compressed and stored for recovery in nearby coastal areas. Likewise, the vessel aims to recycle, by the same process, waste generated on the coast, such as sludge from city and hotel sewage treatment plants, remains of materials, slaughterhouse processing plants and fish processing plants, among others.

The present invention, therefore, may have various configurations in order to adapt to any area of action in which there are plants that prevent the correct development of the usual activity of the area, such as the tourism, fishing, shipping or transport industries. In addition, it can be both a passive and active cleaning system and can be adapted in configuration and size to any environment.

The different objects of the present invention are achieved by the vessel of claim 1. Particular and/or preferred solutions of the present invention are described in the dependent claims.

Throughout the description and claims the word "comprises" and its variants are not intended to exclude other technical features, additives, components or steps. For persons skilled in the art, other objects, advantages and characteristics of the invention will emerge in part from the description and in part from the practice of the invention. The following examples and drawings are provided by way of illustration, and are not intended to limit the scope of protection sought by the claims.

### Brief description of the drawings

Next, a series of drawings are described in a very brief manner which help to better understand the invention and which expressly relate to an embodiment of said invention that is presented as a non-limiting example thereof.
Figure 1 shows a schematic view of the system of collection and pre-treatment of plant remains in coastal areas.
Figure 2 shows a schematic view of the vessel for control of aquatic plant species in seas, rivers and lakes object of the present invention.
Figure 3 shows a detailed view of the bow of the vessel for control of aquatic plant species in seas, rivers and lakes object of the present invention.
Figure 4 shows a schematic view of the internal operation of the vessel for control of aquatic plant species in seas, rivers and lakes object of the present invention.
Figure 5 shows a schematic view of the bow of the vessel (100).

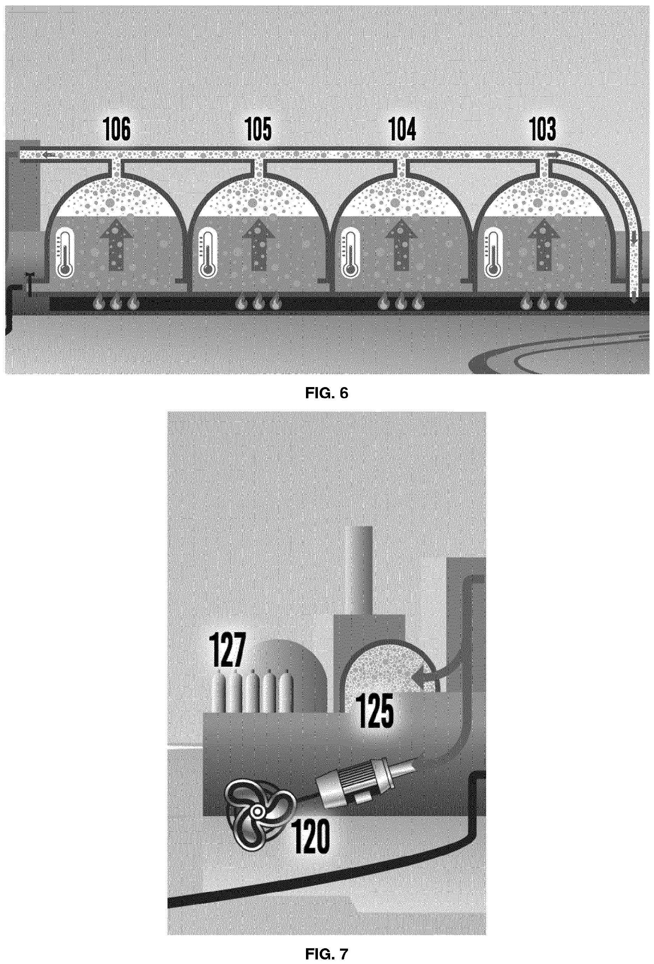

Figure 6 shows a view of the digesters and heating systems.
Figure 7 shows a view of the storage motorization system for the unused methane gas and its subsequent compression.

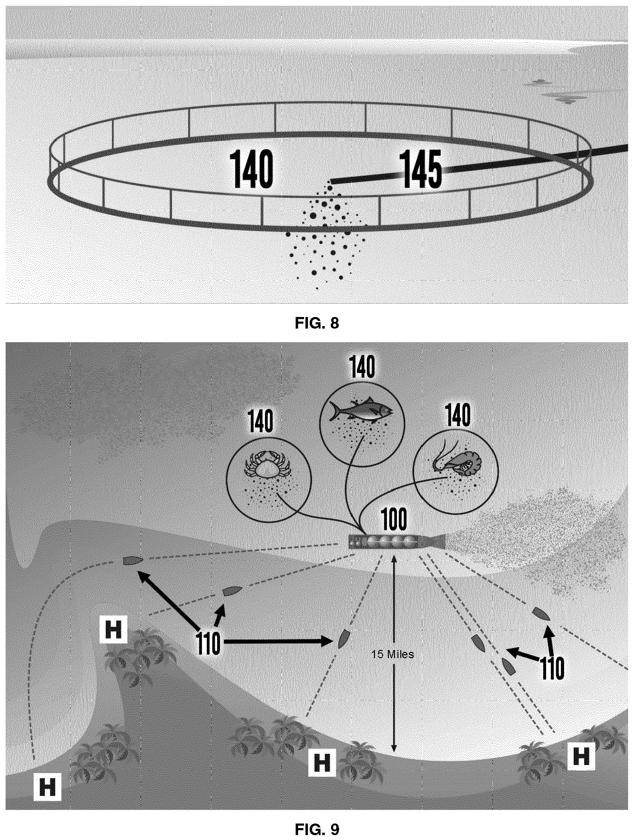

Figure 8 shows the fish farm adjacent to the vessel
Figure 9 shows a schematic view of the positioning of the vessel object of the present invention with respect to the coast where the different hotels that it would serve in a practical example are located.

### Detailed description of an embodiment of the invention

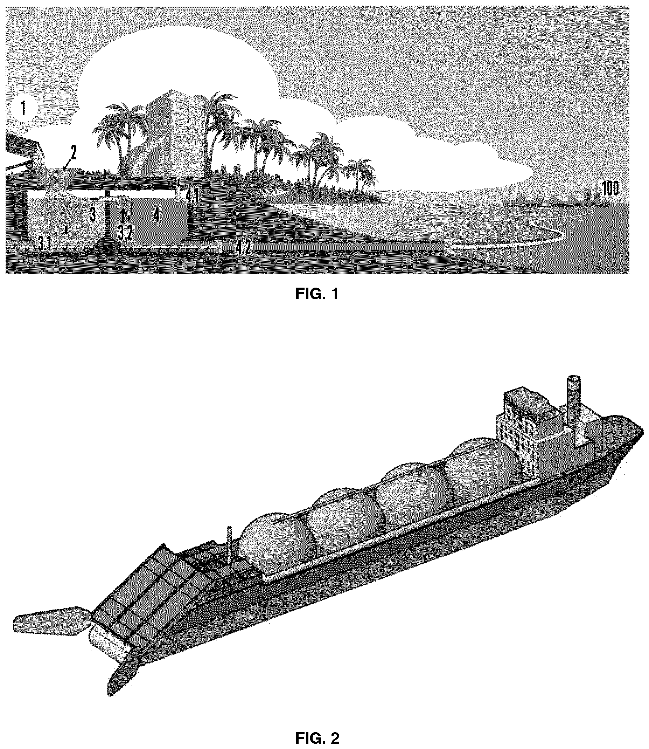

As can be seen in figure 1, the method of control of aquatic plant species begins with the collection on land of aquatic plant species. For example, if we think of the sargassums so common in warm-water seas, such as the Caribbean, this collection is carried out on the beach itself, so the plant species are very mixed with the beach sand. This collection is carried out by mechanical means 1 that deposit the plant residue together with the sand in a hopper 2 that passes to a first tank 3, where the sand is separated from the plant residue by washing and shaking them in said first tank 3. The sand, due to its greater density, precipitates at the bottom of the first tank 3 and through mechanical means 3.1, for example, a worm screw, will be returned to the beach.

This structure has the advantage that it facilitates the collection, since it does not matter that the plant remains arrive mixed with the sand and, in addition, minimizes the environmental impact on the beach, because the sand that is extracted mixed with the plant remains will be returned practically immediately.

In a second reservoir 4, adjacent to the first reservoir 3, the plant remains will be removed, for example, by suction 3.2. In this second reservoir 4, the plant remains are mixed with the used grey water of the hotels and/or nearby towns that are poured 4.1 over this second reservoir 4 to: (i) contribute to the fermentation and decomposition of the plant remains; (ii) complete the purification of the waters offshore in the vessel that is the object of the present invention, thus avoiding any discomfort and/or bad smell to the inhabitants and guests of the coastal areas. Finally, from this second tank 4, and through extraction means 4.2, the plant species mixed with the grey waters will be sent to the vessel 100 object of the present invention.

Parallel to the process described for figure 1, aquatic plant species are also controlled offshore by the vessel 100 schematically shown in figures 2 to 4. As seen in these figures, the vessel 100 is capable of extracting the aquatic plant species and hoisting them on board by means of a mechanized elevation belt 101. These plant species can be invasive or annoying species, crushing them where, subject to the time necessary to the action of heat, their properties are modified, destroying their plant structure and preventing them from reproducing and being returned to the sea as nutrients even in farms adjacent to the vessel 100, thus complying with the biological cycle and control of the invasive species.

Thanks to the vessel of the invention, two basic by-products can be obtained: (a) food for the microorganisms that are at the base of the marine food chain; and (b) the plant debris passes through a cooking chamber 102 and a plurality of digesters 103 to 106 in the same way as in a biogas plant, where at high temperatures methane gas is extracted allowing to: (i) continue feeding the combustion means of the cooking chamber 102 and the digesters 103 to 106; (ii) use it as fuel in an electric cogeneration plant for operating the vessel 100 and the electrical subsystems thereof; and (iii) store 125 and compress 127 the excess methane gas for use on land, for example, in hotels or homes.

The cooking chamber 102 in a practical embodiment also receives the plant remains mixed with the grey waters coming from the coast, as described in reference to figure 1.

In a particular embodiment, the mechanized elevator belt 101 may be provided with floats so that the depth of the extractor belt can be adjusted with respect to the surface. In another particular embodiment, the elevator belt 101 may have mechanisms in front of it that bring the floating plants located on both sides closer to it so that they are aligned towards the centre, improving the cleaning efficiency.

The vessel 100 comprises, as mentioned above, a crusher mechanism 101a arranged at the top of the elevator belt 101. This crushing mechanism can be of any of the types known in the state of the art, such as fixed or oscillating blades or hammers mounted on rotors, so that the extracted materials are fragmented and their fermentation and decomposition is faster. In another practical embodiment, the shredder mechanism 101a may be at the same level as the water surface, such that the shredded debris is sent to the subsequent modules of the vessel 100 mixed with water.

The collected and crushed plant remains pass to a cooking boiler 102 - which in a non-limiting practical embodiment is hermetically sealed - so that the process of cooking and destruction of the reproductive capacity of the plants is faster and more effective. This cooking will also generate steam that can drive a steam turbine that, in turn, moves an electric generator. The steam may also be used to generate the heat required for cooking the extracted and crushed plant debris, as well as the heat required for the digesters 103 to 106.

The collected plant remains, crushed and already subjected to high temperatures, is deposited in digesters 103 to 106 of large dimensions with a controlled temperature -between 40° and 70°- and equally controlled pressure conditions, in such a way that methane gas can be extracted from said remains. The digesters 103 to 106 in a practical embodiment, will be provided with motorized agitators to optimize the generative yield of the gas. The methane gas obtained as a result of the fermentation and putrefaction of the collected and degraded plant remains will be used as fuel for any type of thermal engine, either by pistons or by gas turbines.

The gas obtained in the process performed in the vessel of the invention, in a particular embodiment, may be compressed 125 and stored 127 to be consumed subsequently when no plant remains are extracted or to be used as a commercial product to be used on land, and where the heat produced by the compression of the gas is used to heat the digesters (103 to 106). It can also be used for the internal services of the ship 120.

The vessel 100 in a particular embodiment comprises a laboratory capable of evaluating the complete rotting and corresponding sterilization of the collected plant remains and the characteristics of the gases obtained and the quality of the digested materials to return them to the sea, river or lake with full guarantees. Thus, the decomposed plant remains can be returned to the water 145 to serve as food in fish farms 140 or in areas with wild fish stock.

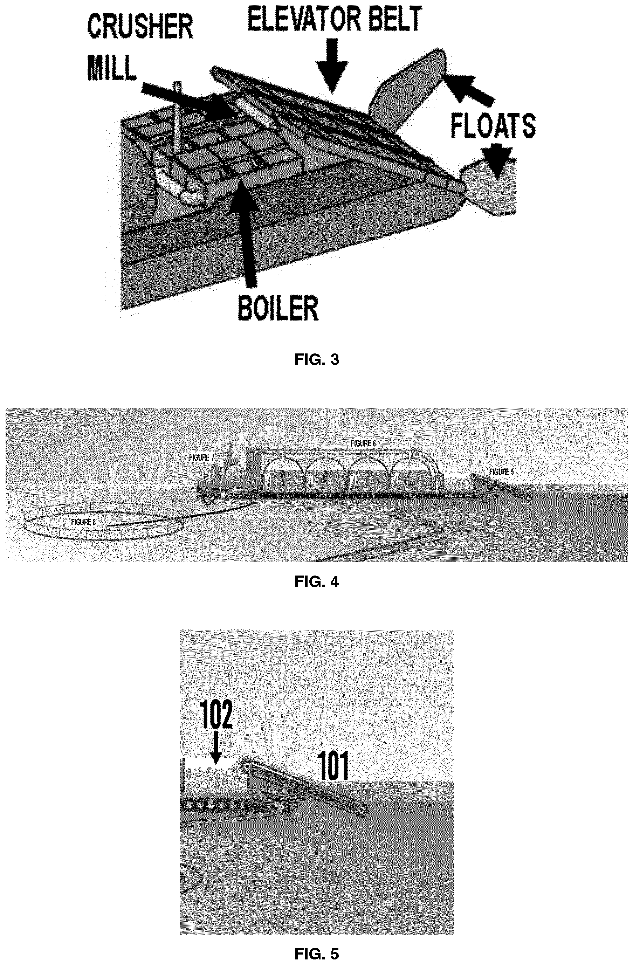

In another practical embodiment, as shown in figure 5, the vessel 100 of the invention is a mother-type vessel that has associated smaller vessels 110 with less draught configured to collect vegetables in areas where the draught is shallower and the density of vegetables is also lower, where the discharge point is in front of the trajectory of the mother vessel, thus ensuring that areas of lower draught or with lower density of plants or invasive species can be efficiently cleaned.

The auxiliary vessels 110 may also serve, in addition, as transport to bring the sargassums or waters coming from hotels or neighbouring urban centres, more efficiently and safely than by floating tubes, thus being able to reach a greater radius of action with the mother ship 100.

In another practical embodiment, the vessel of the invention comprises an aerostatic balloon that reaches sufficient height so that, with a remote-control camera, the surrounding area can be visualized to direct the smaller auxiliary vessels to collect the lower concentrations of algae or invasive species, where this is difficult, impossible or inefficient for the mother vessel.

Once the residue has been fermented, digested, the biogas extracted and the appropriate values verified in the laboratory, it can be poured next to the driving propellers for the optimal diffusion of the residue in the seabed and its best use by the living organisms resident in the ecosystem.

In another practical embodiment, the vessel comprises a rear drag rake configured to bring low-density clusters of vegetables closer to the vessel. Likewise, auxiliary vessels may incorporate a rear rake.

In another practical embodiment, the vessel comprises a compartment for organic materials coming from the inland and susceptible of fermentation, to be processed and decomposed to generate gas, and that after being processed are left inert and tested by the laboratory and, therefore, not harmful to the ecosystem where the intervention is carried out.

In another practical embodiment, two or more vessels may collaborate with each other to collect the plant debris processed by a vessel to continue the fermentation in the same manner as explained above until obtaining the methane gas.

## Claims

1. A vessel for control of aquatic plant species (100) in seas, rivers, and lakes that is configured to:
remove from the surface of the water and hoist to a deck of the vessel (100) a plurality of plant debris from a given aquatic ecosystem by means of an elevator belt (101); and/or
receive from a coastal facility a plurality of aquatic plant debris mixed with grey water from coastal human settlements;
and that **is characterized in that** the remains of aquatic plant species extracted from the surface of the water and/or received from coastal human settlements are deposited in a cooking chamber (102) where they are subjected to controlled temperature conditions configured to modify, ferment and rot said remains, preventing their reproduction.

2. The vessel (100) of claim 1 wherein the vessel (100) comprises a plurality of digesters (103 to 106) configured to extract methane gas from the plant debris already subjected to the action of heat in the cooking chamber (102).

3. The vessel (100) according to any one of the preceding claims wherein the digesters are heated to temperatures between 40°C and 70°C.

4. The vessel (100) of claim 1 or 2, wherein the elevator belt (101) comprises floats configured to adjust the depth of the elevator belt with respect to the surface of the water; and means for aligning the plant debris with respect to the inlet of the elevator belt (101).

5. The vessel (100) of claims 1 or 2 wherein the crushing means (101a) of the elevator belt (101) is disposed at the top of the elevator belt or at the height of the water surface itself.

6. The vessel (100) according to any one of the preceding claims comprising a collection and loading compartment for organic materials from land.

7. The vessel (100) according to any one of the preceding claims comprising a rear drag rake configured to bring closer clusters of vegetables that have not been collected in the first step of the vessel.

8. The vessel (100) according to any one of the preceding claims comprising a plurality of satellite vessels configured for collecting the remains in low draught areas and being able to deposit them on the front of the elevator belt.

9. The vessel (100) according to any one of the preceding claims comprising an aerostatic balloon or drone with a remote-controlled camera configured to visualize the low draught area and supervise and direct the collection of the satellite vessels.

10. The vessel (100) according to any one of the preceding claims wherein the fermented residues, digested and once the methane gas has been extracted, are returned to the water and serve as food to the surrounding fish farms.

11. The vessel (100) according to any one of the preceding claims comprising an on-board laboratory.

12. The method of control of aquatic plant species in seas, rivers and lakes comprising the steps of:
extracting from the surface of the water and hoisting to a deck of the vessel (100) a plurality of plant debris from a given aquatic ecosystem by means of an elevator belt (101) at the front of the vessel configured to extract said plant debris from the aquatic surface; and wherein the elevator belt (101) conveys the debris extracted from the water to grinding means (101a) configured to fragment the debris; and/or
receiving from a coastal facility a plurality of aquatic plant debris mixed with grey water from coastal human settlements;
and that it **is characterized in that** the remains of aquatic plant species extracted from the surface of the water and/or received from coastal human settlements are deposited in a cooking chamber (102) of the vessel (100) where they are subjected to controlled temperature conditions configured to modify, ferment and rot said remains preventing their subsequent reproduction;
and wherein the vessel (100) comprises a plurality of digesters (103 to 106) configured to extract methane gas from the fermented and/or rotten plant remains by the action of heat in the cooking chamber (102).

13. The method according to claim 12 comprising the steps of:
using the methane gas to heat the cooking chamber (102) and/or heat the digesters (103 to 106) or as fuel of the vessel (100); and/or
storing and compressing the methane gas for subsequent use.

14. The method according to claim 12 or 13, wherein the organic residue, already subjected to the action of heat, is used as nutrient for fish and other living beings in fish farms near the vessel (100).
